# EUROPEAN PATENT SPECIFICATION

(11) **EP 1 331 972 B1**
(45) Date of publication and mention of the grant of the patent: **04.07.2007**
(21) Application number: 01982270.9
(22) Date of filing: 10.09.2001
(51) Int. Cl.: A61P 5/00, A61K 31/196

(54) **PHARMACEUTICAL COMPOSITIONS**
PHARMAZEUTISCHE ZUSAMMENSETZUNG
COMPOSITIONS PHARMACEUTIQUES

(30) Priority: 11.09.2000 US 231655 P; 14.09.2000 US 232261 P
(43) Date of publication of application: 06.08.2003
(73) Proprietor: Novartis AG, 4056 Basel (CH); Novartis Pharma GmbH, 1230 Vienna (AT)
(72) Inventor: BATEMAN, Simon, David, Randolph, NJ 07869 (US); GIMONA, Alberto, CH-4052 Basel (CH); HOLINEJ, Jurij, Hackettstown, NJ 07840 (US); HUELS, Jasper, F-68330 Huningue (FR); JAYAWARDENE, Sumedha, Bridgewater, NJ 08807 (US); KARABELAS, Argeris, Jerry, Princeton, NJ 08540 (US); KHALED, Maha, Y., Livingston, NJ 07039 (US); KARNACHI, Anees, Abdulquadar, Hillsborough, NJ 08844 (US); NIC LOCHLAINN, Eimear, Mairin, Castleknock, Dublin 15 (IE); MACERATA, Richard, Summit, NJ 07901 (US); SLOAN, Victor, Scotch Plains, NJ 07076 (US)
(74) Representative: Gros, Florent
(86) International application number: PCT/EP2001/010448
(87) International publication number: WO 2002/020090

(56) References cited:
- WO-A-99/11605
- GOODMAN & GILMAN'S: "THE PHARMACOLOGICAL BASIS OF THERAPEUTICS, 9TH ED." MCGRAW-HILL CO. Section I, General Principles

## Description

### BACKGROUND OF THE INVENTION

This invention relates to the use according to claim 1.

Non-steroidal anti-inflammatory agents are normally administered 2 to 4 times daily. The relatively short half-life of most non-steroidal anti-inflammatory agents, such as aspirin, ibuprofen, naproxen, and diclofenac, means that once and even twice a day administration is not possible, unless the agent is formulated in a controlled or extended release formulation. The relatively large doses needed to achieve once a day treatment of conventional non-steroidal anti-inflammatory agents would also lead to side effects, if given in an immediate release formulation, so that there is a general understanding that once a day administration in an immediate release formulation is unlikely to be achievable.

Surprisingly a compound has been identified which can be employed on a once a day basis and which will not produce an unacceptable level of side effects on such a regimen, and in particular will not cause an unacceptable level of gastric side effects. WO 99/11605, published March 11, 1999 discloses 5-alkyl-2-arylaminophenylacetic acids and derivatives thereof as potent, selective inhibitors of cyclooxygenase-2. We have found that 5-methyl-2-(2'-chloro-6'-fluoroanilino)phenylacetic acid possesses a surprising combination of attributes that make it possible to formulate and use the composition in a unexpected manner. This drug, when administered once a day in an immediate release formulation in an amount between about 200 and about 1200 mg, preferably between about 200 and 800, most preferably about 400 mg, provides effective anti-inflammatory treatment over a 24 hour period, without the use of extended release pharmaceutical formulation excipients and technology.

### SUMMARY OF THE INVENTION

The invention provides use of 5-methyl-2-(2'-chloro-6'-fluoroanilino)phenylacetic acid or a pharmaceutically acceptable salt thereof in the manufacture of a medicament for treating a cyclooxygenase-2 dependent disorder or condition wherein the medicament is for once daily administration.

In another aspect the invention provides a composition for treating a cyclooxygenase-2 dependent disorder or condition comprising 5-methyl-2-(2'-chloro-6'-fluoroanilino)phenylacetic acid or a pharmaceutically acceptable salt thereof, which composition is an immediate release pharmaceutical composition for treatment of said disorder or condition for about 24 hours.

In another aspect the invention is directed to the use according to claim 1 comprising:
administering 5-methyl-2-(2'-chloro-6'-fluoroanilino)phenylacetic acid or a pharmaceutically acceptable salt thereof in an amount effective to treat the disorder for about 24 hours, comprising administering orally once a day to a human in need of such treatment one or more immediate release pharmaceutical compositions comprising 5-methyl-2-(2'-chloro-6'-fluoroanilino)phenylacetic acid or a pharmaceutically acceptable salt thereof.

In another aspect, the invention is directed to a medicament package or composition for treating a cyclooxygenase-2 dependent disorder or condition, the composition comprising:
(a) one or more immediate release pharmaceutical compositions comprising 5-methyl-2-(2'-chloro-6'-fluoroanilino)phenylacetic acid or a pharmaceutically acceptable salt thereof, wherein the one or more immediate release compositions comprise an effective amount of 5-methyl-2-(2'-chloro-6'-fluoroanilino)phenylacetic acid to treat the disorder for about 24 hours; and
(b) printed instructions directing that the one or more immediate release pharmaceutical compositions comprising 5-methyl-2-(2'-chloro-6'-fluoroanilino)phenylacetic acid be administered orally once a day.

### DETAILED DESCRIPTION OF THE INVENTION

The present invention is directed to a composition for the treatment of cyclooxygenase-2 mediated diseases, the composition comprising a cyclooxygenase-2 inhibiting compound characterized by:
(a) high potency for the inhibition of cyclooxygenase-2 as measured by the ability of a single therapeutic dose of the compound to provide relief from osteoarthritis pain;
(b) a half-life 3 to 6 hours; and
(c) a high degree of specificity for inhibiting cyclooxygenase-2 in preference to cyclooxygenase-1 as measured by *in vitro* cellular assays as described in WO 99/11605.

One such compound is 5-methyl-2-(2'-chloro-6'-fluoroanilino)phenylacetic acid. 5-methyl-2-(2'-chloro-6'-fluoroanilino)phenylacetic acid, its utility and methods for its synthesis are disclosed in WO 99/11605.

Thus, the present invention is directed to a composition for treating a cyclooxygenase-2 dependent disorder or condition comprising one or more immediate release pharmaceutical compositions comprising 5-methyl-2-(2'-chloro-6'-fluoroanilino)phenylacetic acid or a pharmaceutically acceptable salt thereof, the one or more immediate release compositions comprising an effective amount of 5-methyl-2-(2'-chloro-6'-fluoroanilino)phenylacetic acid to treat the disorder for about 24 hours and printed instructions directing that the one or more immediate release pharmaceutical compositions comprising 5-methyl-2-(2'-chloro-6'-fluoroanilino)phenylacetic acid be administered orally once a day. Preferably, the immediate release composition(s) does not comprise sufficient water-insoluble or polymeric components to impart extended release characteristics to the composition

As discussed in WO 99/11605, a genus of compounds, including 5-methyl-2-(2'-chloro-6'-fluoroanilino)phenylacetic acid, is useful for the relief of pain, fever and inflammation of a variety of conditions including rheumatic fever, symptoms associated with influenza or other viral infections, common cold, low back and neck pain, dysmenorrhea, headache, including migraine headache, toothache, sprains and strains, myositis, neuralgia, synovitis, arthritis, including osteoarthritis and rheumatoid arthritis, degenerative joint diseases, gout and ankylosing spondylitis, bursitis, bums, and injuries following surgical and dental procedures.

By virtue of its high cyclooxygenase-2 (COX-2) inhibitory activity and/or its selectivity for inhibiting COX-2 over cyclooxygenase-1 (COX-1), 5-methyl-2-(2'-chloro-6'-fluoroanilino)phenylacetic acid is also useful as an alternative to conventional non-steroidal anti-inflammatory drugs (NSAID'S) particularly where such NSAIDS may be contra-indicated such as in patients with peptic ulcers, gastritis, regional enteritis, ulcerative colitis, diverticulitis or with a recurrent history of gastrointestinal lesions; GI bleeding, coagulation disorders including anemia such as hypoprothrombinemia, haemophilia or other bleeding problems (including those relating to reduced or impaired platelet function); kidney disease (e.g. impaired renal function); those prior to surgery or taking anticoagulants; and those susceptible to NSAID induced asthma.

The immediate release pharmaceutical compositions for use in the kits and uses of the invention containing the active ingredient may be in a form suitable for oral use, for example, as tablets, troches, lozenges, aqueous or oily suspensions, dispersible powders or granules, emulsions, hard or soft capsules, or syrups or elixirs. The pharmaceutical compositions useful in the practice of the invention are for oral administration and are "immediate release" dosage forms. That is, the pharmaceutical compositions useful in the practice of the invention have neither the pharmacokinetic nor physical characteristics of extended release pharmaceutical dosage forms. Thus, a pharmaceutical composition useful in the practice of the invention, if in solid form, will disintegrate or dissolve rapidly, preferably within one hour of administration, and administration of a pharmaceutical composition useful in the practice of the invention will result in a rapid rise in the blood plasma concentration of 5-methyl-2-(2'-chloro-6'-fluoroanilino)phenylacetic acid. Preferably, the blood plasma concentration of 5-methyl-2-(2'-chloro-6'-fluoroanilino)phenylacetic acid will reach a maximum within two to six hours after oral administration and will then fall rapidly due to the relatively short (3 to 6 hour) half life of 5-methyl-2-(2'-chloro-6'-fluoroanilino)phenylacetic acid.

Non-immediate release drug formulations, which are not within the scope of the present invention or used therein, include, *inter alia,* delayed release and sustained release formulations. Sustained release formulations may be further subdivided into prolonged release and controlled release formulations. Delayed release systems are those that use repetitive, intermittent dosing of a drug from one or more immediate-release units incorporated into a single dosage form. Examples of delayed release formulations include repeat-action tablets and capsules, and enteric-coated tablets where timed release is achieved by a barrier coating. Delayed release formulations do not produce or maintain uniform blood plasma concentrations of drug, but rather produce intermittent peaks and troughs in the blood plasma concentration of a drug, which are both desirably within the therapeutic range for the drug.

Sustained release drug formulations include drug formulations that achieve slow release of a drug over an extended period of time. If a sustained release formulation can maintain a constant drug concentration in the blood plasma, it is referred to herein as a "controlled release" formulation. If it does not maintain a constant concentration of drug in the blood plasma, but maintains the concentration of the drug in the therapeutic range for a longer period of time than would be achievable with an immediate release formulation, it is referred to herein as a "prolonged release" formulation. Thus, controlled release formulations maintain a relatively constant, peak blood plasma concentration of drug over an extended period of time, typically twelve to twenty four hours; the compositions of the present invention do not.

Typically, sustained release oral dosage formulations are based on a diffusion system, a dissolution system, and osmotic system, or an ion-exchange system.

In diffusion systems, the release rate of the drug is determined by its diffusion through a water-insoluble polymer. There are two types of diffusion devices: reservoir devices, in which a core of drug is surrounded by a polymeric membrane, and matrix devices, in which dissolved or dispersed drug is distributed uniformly throughout an inert polymeric matrix. Typical methods used to make reservoir-type devices include microencapsulation of drug particles and press-coating of whole tablets or particles. Generally, particles coated by microencapsulation form a system where the drug is contained in the coating film as well as in the core of the microcapsule. Some materials typically used as the water-insoluble coating, alone or in combination, are hardened gelatin, methyl or ethylcelluloses, polyhydroxymethacrylate, hydroxypropylcellulose, polyvinylacetate, and waxes.

Matrix devices are typically made by mixing drug with matrix material and then compressing the mixture into tablets. When using wax matrices, drug is generally dispersed in molten wax, which is then congealed, granulated, and compressed into cores. Matrix systems typically have a priming dose of drug coated onto the drug-matrix core. The major types of materials used in the preparation of matrix devices are insoluble plastics, hydrophilic polymers and fatty compounds. Plastic matrices include methyl acrylate-methyl methacrylate, polyvinyl chloride and polyethylene. Hydrophilic polymers include methylcellulose, hydroxypropylmethylcellulose and sodium carboxymethylcellulose. Fatty compounds include waxes such as carnauba wax and glyceryl tristearate.

Most dissolution type sustained release formulations are either encapsulated dissolution systems or matrix dissolution systems. Encapsulated dissolution formulations can be prepared either by coating particles or granules of drug with varying thicknesses of slowly soluble polymers or by microencapsulation. A common method of microencapsulation is coacervation, which involves the addition of a hydrophilic substance to a colloidal dispersion. The hydrophilic substance, which coats the suspended particles, can be selected from a wide variety of natural and synthetic polymers including shellacs, waxes, starches, cellulose acetate phthalate (or butyrate) or polyvinylpyrrolidone. Once the coating material dissolves, all of the drug inside the microcapsule is available immediately for dissolution and absorption, allowing drug release to be controlled by adjusting the thickness and dissolution rate of the coat. If three or four coating thicknesses are used in the microcapsules that comprise a formulation, drugs will be released at different, predetermined times to give a delayed-release, pulsatile effect. If a spectrum of thicknesses is employed, a more constant blood concentration of the drug can be achieved. Encapsulated particles can be compressed into tablets or placed into capsules.

Matrix dissolution sustained release formulations are prepared by preparing particles comprising drug and slowly soluble polymer particles. Such particles can be prepared by congealing drug with a polymer or wax and spray-congealing the particles or by cooling the drug-coating mixture and screening it. Alternatively, an aqueous dispersion method can be used, where a drug-polymer mixture is sprayed or placed in water and the resulting particles are collected. The drug-polymer particles are then compressed into tablets.

Formulations that rely on osmotic gradients have also been used to provide sustained release of drug. Typically, such formulations involve a membrane, permeable to water but not drug, that surrounds a core of drug. The membrane has a small delivery aperture. Water flows through the semipermeable membrane, dissolves drug, which is then pumped out of the formulation through the delivery aperture. Materials that can be used as a semipermeable membrane are polyvinyl alcohol, polyurethane, cellulose acetate, ethylcellulose, and polyvinyl chloride.

The immediate release formulations useful in the practice of the invention are intended for oral use and may be prepared according to any method known to the art for the manufacture of immediate release pharmaceutical compositions. Such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example, magnesium stearate, stearic acid or talc. The excipients cannot be water soluble, water insoluble, or water permeable polymers or waxes where such water soluble, water insoluble, or water permeable polymers or waxes are present in an amount sufficient to impart a sustained release property to the formulation. In a most preferred embodiment, the immediate release pharmaceutical composition is a tablet.

5-methyl-2-(2'-chloro-6'-fluoroanilino)phenylacetic acid has surprisingly been found to possess a duration of action in humans of sufficient length that a single oral dose of between about 200 and about 1200 mg, preferably between about 200 and 800, and most preferably about 400 mg of drug per day in an immediate-release formulation will provide effective safe anti-inflammatory treatment over a 24 hour period. Without being bound by theory, clinical data suggest that the effect of 5-methyl-2-(2'-chloro-6'-fluoroanilino)phenylacetic acid on pain is not directly related to plasma concentration, but rather may be governed by the concentrations of drug in an effect compartment. The relatively constant pain reduction reported by patients receiving a single daily dose of 5-methyl-2-(2'-chloro-6'-fluoroanilino)phenylacetic acid indicates that the response is not greatly influenced by frequency of dosing (e.g., twice daily dosing with 200 mg versus once daily dosing with 400 mg). Such an agent is particularly useful in the treatment of chronic indications, such as rheumatoid arthritis and osteoarthritis as well as Alzheimer's Disease and prophylaxis of colon cancer, where the drug needs to be taken every day for the duration of a subject's life, because compliance is much easier with once a day dosing.

Oral dosage levels for 5-methyl-2-(2'-chloro-6'-fluoroanilino)phenylacetic acid are of the order of between about 200 and about 1200 mg per patient per day. In a preferred embodiment, the effective amount is between about 200 and about 800 mg. In a more preferred embodiment, the effective amount is between about 200 and about 600 mg. In an even more preferred embodiment, the effective amount is between about 200 and about 400 mg. In the most preferred embodiment, the effective amount is about 400 mg.

The amount of drug that may be combined with the carrier materials to produce a single dosage form will vary depending upon the size and weight of the recipient, the body composition of the recipient, and the particular mode of administration. For example, a formulation intended for oral administration by human recipients may contain between about 50 and about 1200 mg of agent compounded with an appropriate and convenient amount of carrier material which may vary from about 5 to about 95 percent of the total composition. Dosage unit forms may typically contain drug in amounts of 50, 100, 200, 300, 400, 600 or 800 mg. In one embodiment, the immediate release pharmaceutical composition comprises between about 50 and about 1200 mg of the 5-methyl-2-(2'-chloro-6'-fluoroanilino)phenylacetic acid or a pharmaceutically acceptable salt thereof. In a preferred embodiment, the immediate release pharmaceutical composition comprises between about 50 and about 600 mg of the 5-methyl-2-(2'-chloro-6'-fluoroanilino)phenylacetic acid or a pharmaceutically acceptable salt thereof. In a yet more preferred embodiment, the immediate release pharmaceutical composition comprises between about 50 and about 400 mg of the 5-methyl-2-(2'-chloro-6'-fluoroanilino)phenylacetic acid or a pharmaceutically acceptable salt thereof. In the most preferred embodiment, the immediate release pharmaceutical composition comprises about 400 mg of the 5-methyl-2-(2'-chloro-6'-fluoroanilino)phenylacetic acid or a pharmaceutically acceptable salt thereof. In a preferred embodiment, the immediate release composition comprises a capsule or tablet. In the most preferred embodiment, the immediate release pharmaceutical formulation comprises a tablet.

In another aspect, this invention provides a composition of 5-methyl-2-(2'-chloro-6'-fluoroanilino)phenylacetic acid or a pharmaceutically acceptable salt thereof having a drug loading of 60% to 90% by weight based on the weight of the composition.

In a further aspect, this invention provides an immediate release tablet comprising 400 mg of 5-methyl-2-(2'-chloro-6'-fluoroanilino)phenylacetic acid or a pharmaceutically acceptable salt thereof, wherein the tablet comprises between about 60% and about 70% of 5-methyl-2-(2'-chloro-6'-fluoroanilino)phenylacetic acid or a pharmaceutically acceptable salt thereof by weight. The immediate release tablet may comprise about 65% of 5-methyl-2-(2'-chloro-6'-fluoroanilino)phenylacetic acid or a pharmaceutically acceptable salt thereof by weight.

It will be understood, however, that the specific dose level for any particular patient will depend upon a variety of factors including the age, body weight, general health, sex, diet, time of administration, rate of excretion, drug combination and the type and severity of the particular disease undergoing therapy. For many patients, a once daily dosage range of between about 200 and about 1200 mg per day, preferably between about 200 and about 400 mg per day, most preferably about 400 mg per day is indicated.

For long term therapy, such as in the treatment of chronic diseases including rheumatoid arthritis, osteoarthritis, Alzheimer disease, or prophylaxis of colon cancer, a once daily dosage of between about 200 and about 1200 mg per day, preferably between about 200 and about 800 mg per day or between about 200 and about 600 gm per day, most preferably between about 200 and about 400 mg per day, is indicated. More particularly, for the treatment of osteoarthritis, a dosage of between about 200 and about 400 mg per day is preferred, whereas for the treatment of rheumatoid arthritis, between about 400 and about 1200 mg per day is preferred. For the treatment of non-chronic indications such as headache or post-operative swelling and pain, between about 200 and about 400 mg per day is preferred.

The invention provides in a further aspect a highly compressed tablet with a high drug loading. The tablet may be small in dimension e.g. 10 to 20 mm in diameter, preferably 15 to 20 mm, most preferably 17 to 18 mm; 5 to 10 mm in width, preferably 6.5 to 7.5 mm. The thickness of the tablet is from 4 to 8 mm, preferably 4.5 to 6.5 mm, most preferably 5.8 mm. Compression forces of between 10 to 20 kilo Newtons are used to prepare the compressed tablet. Benefits of this high drug loading include improved bioavailability, release characteristics and compliance.

The medicament package comprises a composition and printed instructions directing that one or more pharmaceutical compositions comprising 5-methyl-2-(2'-chloro-6-fluoro-anilino)phenylacetic acid or a pharmaceutically acceptable salt thereof be administered orally once a day.

Following is a description by way of example only of compositions of the invention.

### Example 1: Preparation of Formulation

**Table 1**

| **Ingredient** | **Amount per 200 mg tablet batch (kg)** |
|---|---|
| **Core** | |
| **Granulation** | |
| 5-methyl-2-(2'-chloro-6'-fluoroanilino)phenylacetic acid drug substance | 50** |
| Microcrystalline cellulose, NF (PH 101) | 12.85 |
| Lactose monohydrate, NF | 11.65 |
| Croscarmellose sodium, NF | 1 |
| Povidone, USP | 4 |
| Titanium dioxide, USP | 2 |
| Water, purified ***, USP | 20.375 |
| **Extra-granular Phase** | |
| Microcrystalline cellulose, NF (PH 102) | 13 |
| Croscarmellose sodium, NF | 3 |
| Titanium dioxide, USP | 2 |
| Magnesium stearate, NF | 0.5 |
| **Coating** | |
| Opadry white | 2.801 **** |
| Opadry yellow | 2.0 **** |
| Opadry red | 0.4 **** |
| Opadry black | 0.0504 **** |
| Water, purified ***, USP | 29.758 **** |

| | |
|---|---|
| ** The weight of drug substance is taken with reference to the dried substance (100 per cent) on the basis of the assay value (factorization). The difference in weight is adjusted by the amount of microcrystalline cellulose used. *** Removed during processing. **** Includes a 50 % excess for loss during the coating process. | |

Table 1, above, sets out the formula for a batch of approximately 250,000 immediate release film-coated tablets of 5-methyl-2-(2'-chloro-6'-fluoroanilino)phenylacetic acid. To make the tablets, titanium dioxide is dispersed in water, followed by the addition of povidone and mixing for 20 minutes to make a povidone/titanium dioxide suspension. The drug substance, lactose, microcrystalline cellulose, and croscarmellose are mixed in a high shear mixer (e.g., a Collette Gral) for 5 minutes to form a drug mixture. The drug mixture is granulated in the high shear mixer with the povidone/titanium dioxide suspension. The suspension is pumped at a rate of 3 kg/min into the drug mixture. The resulting mixture is mixed an additional 90 seconds after all the suspension is added. The wet granulation is dried in a fluid bed dryer, using an inlet air temperature of 50°C. The residual water target is 3.5 % (with a permissible range of 2.5 - 4.5 %). The dried granulation is passed through a screen using a mill (oscillator) and a 30 mesh screen. The previous steps are repeated to make a second granulation.

The extra-granular phase titanium dioxide is passed through a 60 mesh hand screen. The dry granulations are mixed with the extra-granular phase microcrystalline cellulose, croscarmellose sodium and titanium dioxide in a twin shell mixer for 300 revolutions to form a penultimate mixture. Magnesium stearate is passed through a 60 mesh hand screen and is mixed with the penultimate mixture in a twin shell mixer for 50 revolutions to form a tableting mixture. The tableting mixture is pressed into tablets using a tablet press and oval punches.

The coating powders (Opadry) are mixed with purified water to make a 15 % w/w coating suspension. The tablets are film coated with the coating suspension in a coating pan using 60 °C to 75 °C inlet air temperature. Table 2 sets out the contents of a 200 mg 5-methyl-2-(2'-chloro-6'-fluoroanilino)phenylacetic acid film-coated tablet.

**Table 2**

| **Ingredient** | **Theoretical amount [mg]** | **Function** |
|---|---|---|
| **Core** | | |
| 5-methyl-2-(2'-chloro-6'-fluoroanilino)phenylacetic acid drug substance | 200 | Active substance |
| Microcrystalline cellulose (PH 101) | 51.4 | Filler |
| Lactose | 46.6 | Filler |
| Povidone | 16 | Binder |
| Titanium dioxide | 8 | Color |
| Croscarmellose sodium | 4 | Disintegrant |
| Water, purified * | Q.S. | Granulating liquid |

| **Extragranular phase** | | |
|---|---|---|
| Microcrystalline cellulose (PH 102) | 52 | Filler |
| Croscarmellose sodium | 12 | Disintegrant |
| Titanium dioxide | 8 | Color |
| Magnesium stearate | 2 | Lubricant |
| Core weight | **400** | |

| **Coating** | | |
|---|---|---|
| Opadry white (00F18296) | 7.4676 | Color |
| Opadry yellow (00F12951) | 5.3312 | Color |
| Opadry red (00F15613) | 1.0668 | Color |
| Opadry black (00F17713) | 0.1344 | Color |
| Water, purified * | Q.S. | Coating solvent |
| **Total weight** | **414** | |

| | | |
|---|---|---|
| * removed during processing | | |

In addition, the tablet formulations may contain 5-methyl-2-(2'-chloro-6'-fluoroanilino)benzyl alcohol and/or 5-methyl-2-(2'-chloro-6'-fluoroanilino)benzoic acid in an amount between about 0.01 and 2% by weight, more specifically between about 0.1 and 1%. Thus, in a further embodiment the present invention is directed to a pharmaceutical composition comprising an effective amount of 5-methyl-2-(2'-chloro-6'-fluoroanilino)phenylacetic acid and between 0.01 and 2% by weight of 5-methyl-2-(2'-chloro-6'-fluoroanilino)benzyl alcohol.

### Example 2: Activity of 5-methyl-2-(2'-chloro-6'-fluoroanilino)phenylacetic acid in humans

A four week multicenter randomized double-blind double-dummy placebo-controlled parallel group trial (the study) of four dosing regimens of 5-methyl-2-(2'-chloro-6'-fluoroanilino)phenylacetic acid in patients with knee or hip primary osteoarthritis is performed, using diclofenac as a comparator. Patients are male and female, from 18 to 75 years of age, have a clinical and radiological diagnosis of knee or hip osteoarthritis as defined by the American College of Rheumatology criteria, and have a pain intensity of at least 40 mm (Visual Analog Scale) in the affected joint prior to screening for inclusion in the study. 584 patients are randomized to receive either 50, 100, or 200 mg 5-methyl-2-(2'-chloro-6'-fluoroanilino)phenylacetic acid or 75 mg diclofenac administered orally twice a day, or 400 mg 5-methyl-2-(2'-chloro-6'-fluoroanilino)phenylacetic acid administered orally once a day, or placebo. Drug and placebo are administered in capsules. The comparator, diclofenac, is an extended release tablet that is over-encapsulated to match the placebo. The treatment duration is for 28 days.

Pharmacokinetics are performed on 52 of the patients who receive 5-methyl-2-(2'-chloro-6'-fluoroanilino)phenylacetic acid. Blood samples are taken at 0, 0.5, 1, 2, 3, 4 and 6 hours following administration of 5-methyl-2-(2'-chloro-6'-fluoroanilino)phenylacetic acid. Plasma concentrations of 5-methyl-2-(2'-chloro-6'-fluoroanilino)phenylacetic acid are measured by an LC/MS assay with a limit of quantitation of 10 ng/ml. Cₘₐₓ and tₘₐₓ AUC (0-6) are determined for day 1 and Cₘₐₓ^{ss} and tₘₐₓ^{ss} AUCτ^{ss}, Cₘᵢₙ^{ss}, Cₐᵥ^{ss}, peak-trough fluctuation (PTF) and Cl/F at steady state are determined on day 28. The primary efficacy parameter is the most pain experienced over the previous 24 hours on a 100 mm VAS.
Figure 1 is a plot of AUCτ on day 28 of the study versus the dose.
Figure 2 is a plot of Cₘₐₓ on day 28 of the study versus dose in the twice daily dosage groups.
Figure 3 is a box plot of plasma trough concentrations of 5-methyl-2-(2'-chloro-6'-fluoroanilino)phenylacetic acid on day 28 of the study.
Figure 4 is a plot of mean plasma concentrations of 5-methyl-2-(2'-chloro-6'-fluoroanilino)phenylacetic acid versus time on day 28 of the study.
Figure 5 is a plot of mean VAS scores versus time on day 0 of the study.
Figure 6 is a plot of mean VAS scores versus time on day 28 of the study.
Figure 7 is a plot of typical mean VAS scores versus plasma concentrations of 5-methyl-2-(2'-chloro-6'-fluoroanilino)phenylacetic acid in the 400 mg once daily dosage group.
Figure 8 is plot of change in VAS scores from the greatest pain in the previous 24 hours versus AUC on day 28 of the study.

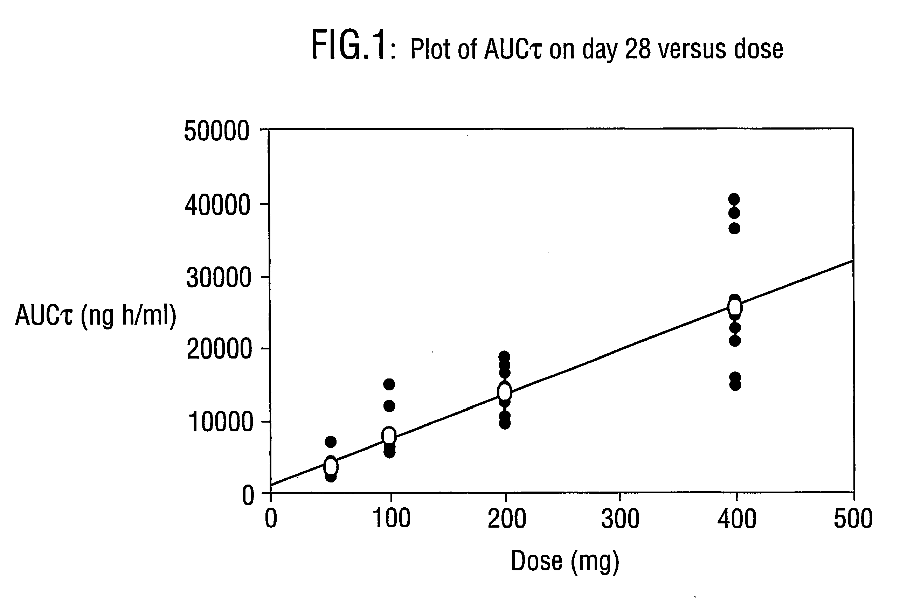

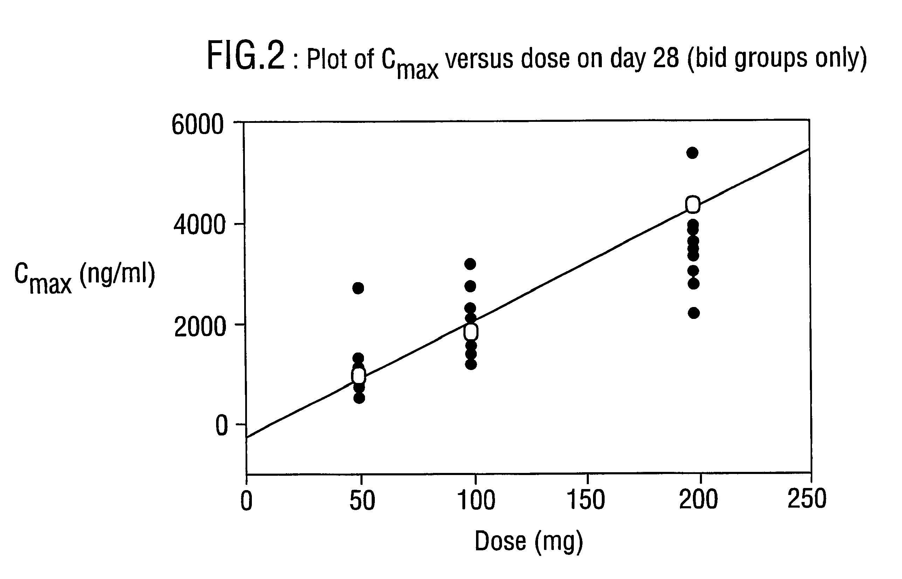

Following multiple oral administration of 5-methyl-2-(2'-chloro-6'-fluoroanilino)phenylacetic acid to osteoarthritis patients at does of 50, 100, and 200 mg twice daily and 400 mg once daily, there is a dose proportional increase in AUC and Cₘₐₓ on day 28 (Figures 1 and 2 and Table 3). The Cₘₐₓ of the 400 mg group was similar to that of the 200 mg twice daily group at steady state (Table 3).

**Table 3**

| | DAY 0 | | | DAY 28 | | | | |
|---|---|---|---|---|---|---|---|---|
| Dose (mg) | Cmax (ng/ml) | AUC₀₋₆ (ng h/ml) | tₘₐₓ(h) | Cₘₐₓ (ng/ml) | tₘₐₓ (h) | AUC₀₋₆ (ng h/ml) | AUCτ (ng h/ml) | CLₛₛ/F (l/h) |
| 50 bid | 914 ± 340 | 2682 ± 960 | 2.0 (0.5:6. 0) | 1008 ± 590 | 2.0 (1.0:3. 0) | 2808 ± 1210 | 3662 ± 1290 | 15.0 ± 4.7 |
| 100 bid | 1787 ± 600 | 5220 ± 1810 | 2.5 (1.0:4. 0) | 1862 ± 630 | 2.0 (0.5:4. 0) | 6008 ± 1890 | 8098 ± 2730 | 13.3 ± 3.3 |
| 200 bid | 4207 ± 2160 | 11730 ± 3570 | 3.0 (0.5:4. 0) | 4378 ±3100 | 2.0 (1.0:3. 5) | 11089 ±3024 | 14029 ±3220 | 15.0 ± 3.5 |
| 400 qd | 5536 ± 1800 | 17245 ± 5280 | 3.0 (1.1:4. 1) | 4788 ± 2640 | 2.0 (1.0:4. 0) | 15310 ± 5590 | 25750 ± 9200 | 17.4 ± 6.1 |

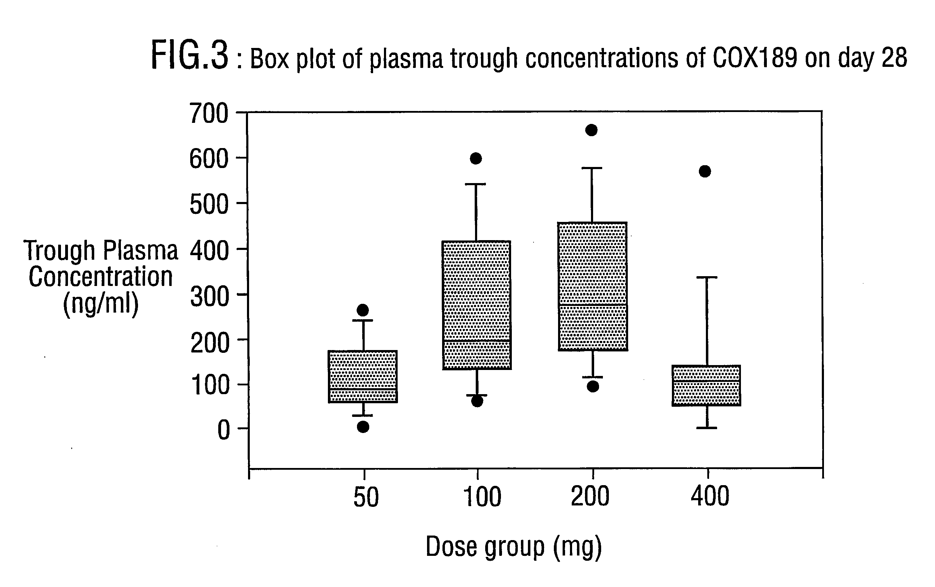

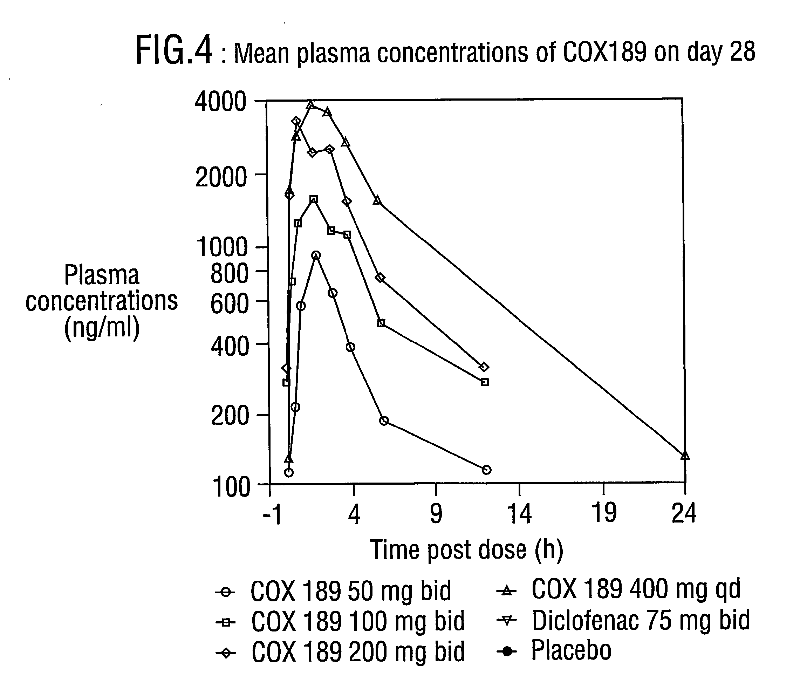

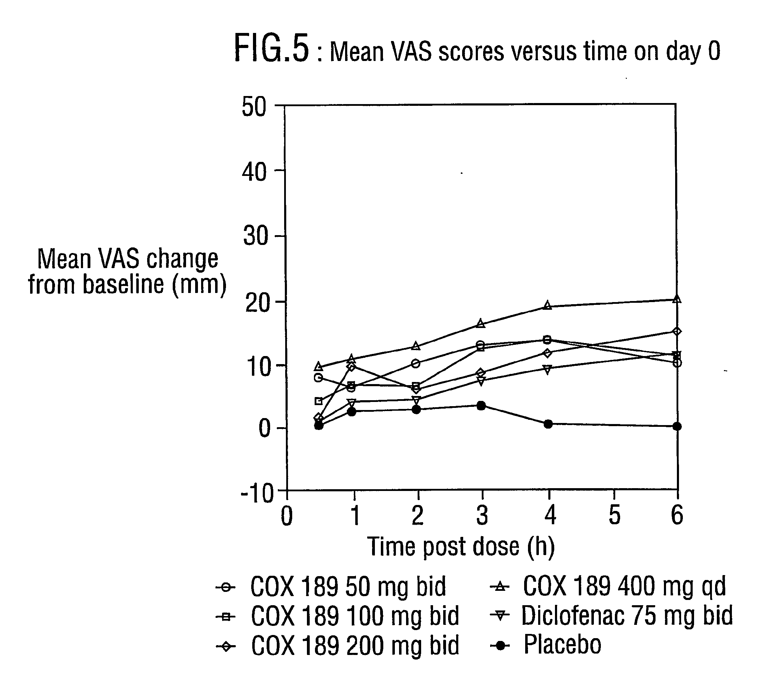

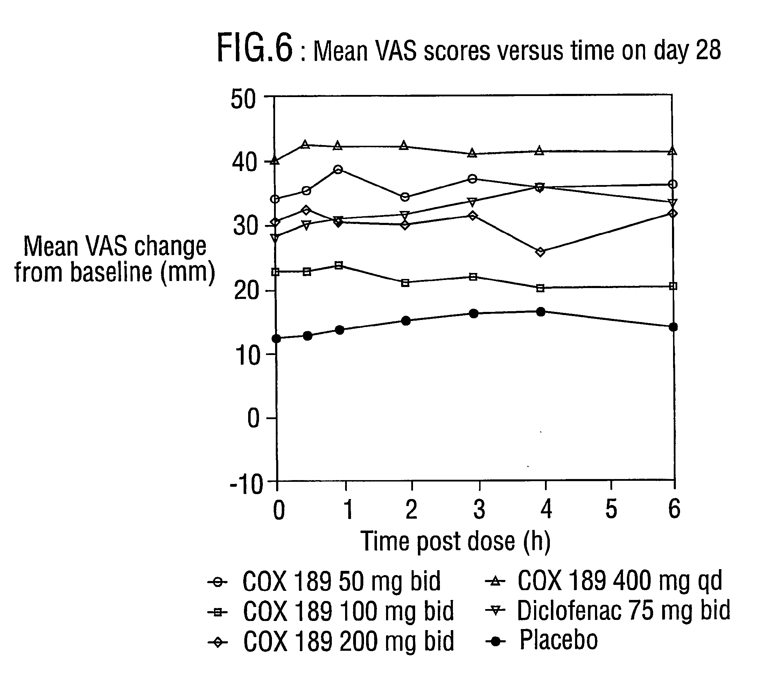

Trough plasma concentrations of 5-methyl-2-(2'-chloro-6'-fluoroanilino)phenylacetic acid are shown in Figure 3. The trough values observed in the twice daily groups increase approximately in proportion to the dose, e.g., the troughs in the 400 mg once daily group is similar to the trough in the 50 mg twice daily group (see also Figure 4). The tₘₐₓ occurs, typically, at 2 to 3 hours. AUC₀₋₆ is similar on day 0 and day 28 (Table 3) suggesting that 5-methyl-2-(2'-chloro-6'-fluoroanilino)phenylacetic acid reaches steady state in plasma rapidly. Plots of VAS pain scores are plotted versus time post dose, on study day 0 (Figure 5) and day 28 (Figure 6). On study day 0, in each dose group, except placebo, there is a reduction in VAS score with time which reaches a maximum at either 4 or 6 hours post dose. On day 28 all dose groups show an increase in the change of VAS from baseline, and mean values for all active dose groups are better than placebo. Unlike the data of day 0, the VAS scores on day 28 are relatively constant with respect to time post dose. Not surprisingly, given the variability in VAS pain measurements, a dose response is not readily apparent. However, the 400 mg once daily dose gave the best mean scores throughout the measurement period. Table 4 summarizes the VAS results at days 7, 14, and 28.

**Table 4**

| | Baseline | Day 7 | Day 14 | Day 28 |
|---|---|---|---|---|
| 50 mg bid | 66.9 (14.0) | -19.5 (19.1)* | -22.6 (20.9)* | -28.6 (24.0)* |
| 100 mg bid | 64.7 (14.3) | -19.2 (21.6)* | -21.2 (22.6)* | -26.3 (23.4)* |
| 200 mg bid | 67.0 (13.0) | -22.0 (20.8)* | -24.1 (22.8)* | -29.6 (26.8)* |
| 400 mg qd | 66.9 (13.0) | -26.0 (22.4)*a,b | -27.5 (23.8)* | -33.2 (24.8)* |
| diclofenac/75 mg | 66.1 (13.7) | -27.9 (22.4)*a,b,c | -30.4 (23.0)* a,b,c | -31.8 (25.0)* |
| placebo | 67.9 (12.7) | -9.7 (17.6) | -13.7 (22.1) | -17.7 (24.5) |

| | | | | |
|---|---|---|---|---|
| * p<0.05 vs placebo at time point; a, p<0.05 vs 50 mg bid; b, p<0.05 vs 100 mg bid; c, p<0.05 vs 200 mg bid | | | | |

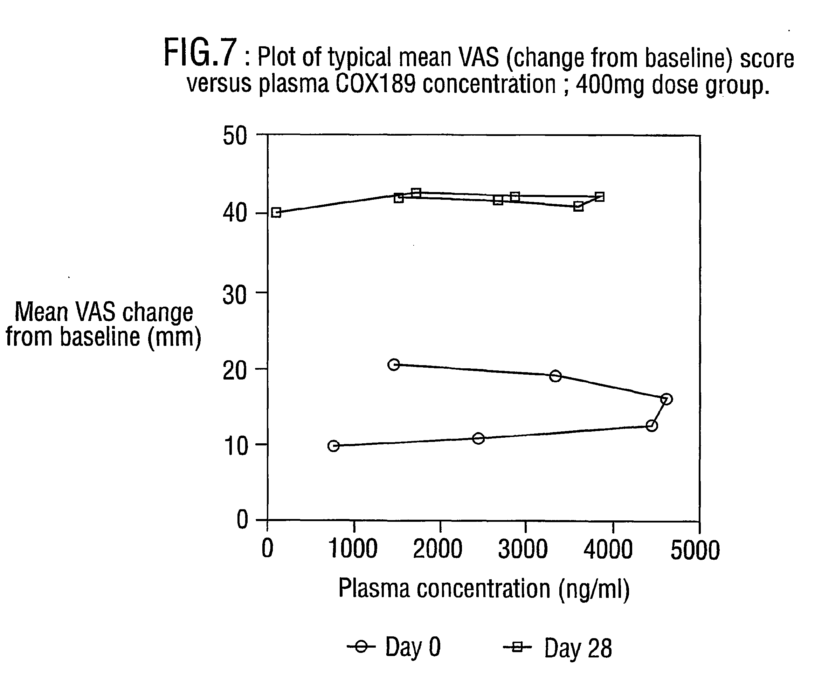

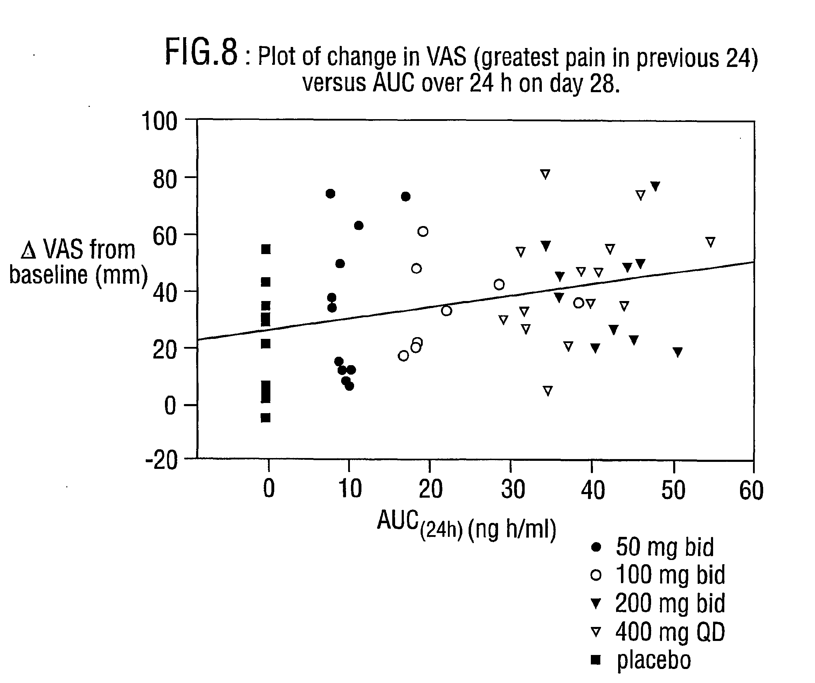

On day 0, a plot of mean plasma concentration versus mean change in VAS from baseline using a once daily 400 mg dose results in an anti-clockwise hysteresis loop (Figure 7). Without being bound by theory, such a phenomenon may result from the action of the drug in an effect compartment. By day 28 the VAS score is relatively constant over the sampling period, and thus there appears to be no direct relationship between plasma concentration and effect. A once daily dose of 400 mg of 5-methyl-2-(2'-chloro-6'-fluoroanilino)phenylacetic acid gives the same or better pain relief as 200 mg twice daily. Plotting the VAS pain measurement over the previous 24 hours (primary end point) on day 28 versus exposure over 24 hours give a positive correlation with r² of 0.12 (Figure 8).

### Example 3: Highly compressed 400 mg drug loading

To develop a high drug loading 400 mg active agent composition, high shear granulation and spray granulation processes are evaluated in parallel. The drug substance is granulated with a povidone solution. Povidone concentrations of 4 % (96% drug loading) and 8 % (92% drug loading) are used. The granulations are dried and milled through a 20 mesh (0.84 mm) (see: Remington: The Science and Practice of Pharmacy, Nineteenth edition, Volume II, p. 1606). Portions of granules that are retained on the 60 mesh (0.25 mm) and 80 mesh (0.175 mm) are mixed and are compressed on a carver press. Round 11 mm flat face beveled edge tooling is used and compression forces ranging from 2 to 10 KN (Kilo newtons) are applied to generate a compression profile. Increase in the tensile strength is evaluated by determining the hardness of the compacts upon crushing. Increasing the force from 2 KN to 10 KN resulted in harder compacts with tensile strengths ranging from 78 N (Newtons) to 137 N. Evaluations are performed in duplicate. The tensile strength of compacts granulated with 8% povidone is slightly higher than compacts granulated with 4% povidone. However, there is no significant difference in hardness values observed between granules that are made with either spray granulation or the high shear granulation.

Further experiments are conducted by processing these granulations into feasible formulations and compressibility is evaluated. Three different drug loading levels are evaluated. Drug loading using 85%, 75%, and 65% active are studied. This is performed for both the high shear and spray granulation processes. The drug substance is granulated with a povidone solution. The granulation is then dried and milled. The following components are added as running powders to the milled granules: microcrystalline cellulose (PH 102), croscarmellose sodium, and magnesium stearate. Blends are compressed on a Beta press using appropriate tooling size and compressibility is studied.

Initially the higher drug load at 85% active is evaluated. Compression profiles that are generated demonstrate that the high shear granulation process results in harder tablets with increasing applied forces. Compression forces greater than 12 kilonewtons (KN) cause lamination of the 85% active tablets. In comparison, lamination is observed at forces higher than 9 KN for the spray granulation batch. Both of the formulations with 85% drug loading result in high friability values in excess of 2% after 200 drops. Friability is a measure of the brittleness of tablets, and is measured by weighing tablets before and after they have been subjected to "dropping", and dividing the difference between tablet weight before dropping and the tablet weight after dropping by the tablet weight before dropping and multiplying by 100. Friability is measured using a friabilitor, which is a rotating drum that, every revolution, drops tablets enclosed therein a distance of 6 inches. Typically, about 20 tablets are used for each friability test. Acceptable friability is defined as less than 1%. Accordingly, compressibility is not satisfactory at 85% drug loading.

Drug loading at 75% and 65% active is evaluated. The high shear batch has better compressibility than batch the spray granulation batch at the forces applied. Tablets laminate upon crushing at 16 KN force for the high shear process as compared to lamination at 12 KN force for the spray granulation batch. Although friability values are still unacceptably high after 500 drops, data from these experiments suggest that 65% drug loading can yield tablets with satisfactory compression properties. High shear granulation with 65% drug loading is selected for further formulation development.

Evaluation of 400 mg tablet formulations was based on a unit weight of cores of 615 mg containing 400 mg of drug substance, which is 65.04% drug load, with povidone as a binder. The disintegrant (croscarmellose sodium) is split equally into intragranular and extragranular portions. The extragranular portion disintegrates tablets into granules and the intragranular portion reduces granules to even finer particles, facilitating dissolution and release. The filler, microcrystalline cellulose (PH 102) is added extragranularly as is magnesium stearate, which is used as a lubricant. Three formulation factors are studied at three different levels and five responses evaluated. These are shown in Table 5.

**Table 5 Formulation variables and responses.**

| **Factors** | **Levels** | **Responses** |
|---|---|---|
| Binder | 4%, 6%, 8% | Compressibility |
| (Polyvinylpyrrolidone, Povidone K30) | | |
| Disintegrant (croscarmellose sodium, Ac-Di-Sol) | 2%, 4%, 6% | Ejection force |
| Lubricant (Magnesium Stearate) | 0.5%, 1 %, 1.5% | Friability (500 drops) |
| | | Disintegration time Dissolution rate |

The tablets are formulated by first mixing the polyvinylpyrrolidone binder with water, followed by addition of the COX189 drug substance and croscarmellose sodium to the povidone solution. This mixture is granulated in a Gral mixer. The resulting granulation is dried in a fluid bed dryer, and is screened over an oscillating 18 mesh screen.Microcrystalline cellulose (Avicel PH-102, NF) is mixed with croscarmellose sodium and the resulting mixture is screened over an 18 mesh screen. The screened mixture is blended with the screened, dried granulation of polyvinylpyrrolidone, drug substance, and croscarmellose sodium. The resulting mixture is then blended with magnesium stearate that has been screened through an 18 mesh screen. The resulting blend is then compressed on a tablet press. An eight run experimental design is generated with two replicate runs (run 7 and 8). Table 6 shows the experimental runs.

**Table 6 Experimental runs**

| **Experiment** | **Binder (%)** | **Disintegrant (%)** | **Lubricant (%)** |
|---|---|---|---|
| 1 | 4 | 6 | 1 |
| 2 | 8 | 2 | 1 |
| 3 | 7.5 | 2 | 1.5 |
| 4 | 4.5 | 6 | 0.5 |
| 5 | 8 | 2.5 | 0.5 |
| 6 | 4 | 5.5 | 1.5 |
| 7 | 6 | 4 | 1 |
| 8 | 6 | 4 | 1 |

The following process conditions are kept similar for all the eight experiments. These include:
Equipment: 10L Gral mixer, GPCG-1 dryer, Frewitt mill, 4 Qt V-blender, Beta Press
Water amount: 18.5% of the sum of drug substance, croscarmellose sodium and povidone weight.

| | |
|---|---|
| Binder addition rate: | 3 minutes 30 seconds for all the batches |
| Granulation time: | between 30 seconds to 2 minutes |
| Drying temperature: | 50°C |
| Residual moisture: | 0.5 to 2% (%LOD) |
| Mesh size: | US standard 18 (1.0 mm) |

Tablet tooling:17 X 6.7 mm Ovaloid with NVTRD debossed on one side and 984 on the other side.

| | |
|---|---|
| Tablet press: | Beta press with gravity feed frame |
| Tablet press speed: | 75-80 RPM |

Tablets are compressed at various forces to generate compression profiles. The compression and ejection forces are monitored during compression using an instrumented tablet press. Friability, disintegration time and dissolution of the cores are also evaluated. Table 7 shows the data at 13-16 KN force for the eight experiments.

**Table 7 Tablet physical characterization data for the experimental runs**

| **Compression force (KN)** | **Hardness (Kp)** | **Ejection force (N)** | **DT mins:secs** | **Dissolution at 15 mins (%)** | **Dissolution at 60 mins (%)** | **Friability (%)** |
|---|---|---|---|---|---|---|
| 14.9 | 19.9 | 590 | 1:48-2:42 | 81.9 | 93.4 | 1.26 |
| 14.8 | 17.0 | 546 | 9:30-10:18 | 73.7 | 85.1 | 1.30 |
| 14.5 | 18.1 | 511 | 8:32-10:13 | 73.1 | 83.9 | 1.03 |
| 15.2 | 19.1 | 648 | 3:05-3:50 | 81.8 | 89.5 | 0.90 |
| 14.6 | 19.2 | 583 | 8:45-9:20 | 67.5 | 87.0 | 0.40 |
| 13.9 | 17.1 | 542 | 2:07-2:28 | 74.6 | 90.0 | 2.45 |
| 13.9 | 19.0 | 526 | 5:15-5:58 | 75.6 | 90.1 | 1.02 |
| 13.6 | 17.9 | 516 | 4:47-5:32 | 78.5 | 87.6 | 0.83 |

Friability is measured after 500 drops, and the acceptable dissolution standard is 70% dissolved in 60 minutes. It is observed that binder and disintegrant had a significant effect on disintegration time, dissolution, hardness and ejection forces. Despite the different levels of binder and disintegrant, all batches pass the dissolution test (Q point of 70% drug released in 60 minutes). Lubricant levels had a significant effect on friability after 500 drops, but had no significant effect on ejection forces. For further optimization, friability is optimized as this response is considered most critical for successful scale-up and developing a robust coated tablet. Friability of less than 1% (preferably around 0.4 - 0.6%) after 500 drops is targeted for optimization. Table 8 lists the constraints on acceptable tablet properties.

**Table 8 Constraints on tablet properties**

| **Responses** | **Constraints** |
|---|---|
| Friability | Between 0.1 % and 0.6 % |
| Hardness | Greater than 15 Kp |
| Disintegration time | Less than 9 minutes |
| Dissolution (after 15 minutes) | 75 % drug released in 15 minutes |
| Ejection force | Less than 1000 N |

**Table 9 Optimized factor levels and predicted responses**

| **Optimized factor levels** | **Predicted responses** |
|---|---|
| Binder 6.55% | Friability 0.5% |
| Disintegrant 4.26% | Hardness 19.7 Kp |
| Lubricant 0.42% | Disintegration time 6 minutes 36 seconds |
| | Dissolution after 15 minutes 75% |
| | Ejection force 634 N |

An optimized formulation is manufactured at with 6.55% binder, 4.26% disintegrant, and 0.42% lubricant levels, which yielded the properties set out in Table 10 below.

**Table 10 Observed and predicted values of the properties for optimized formulation at 14 KN compression force**

| **Properties** | **Observed** |
|---|---|
| Friability | 0.72 % |
| Hardness | 18.3 Kp (17.4 - 19.4) |
| Ejection force | 688 N |
| Disintegration time (mins:secs) | 6:54 - 7:28 |
| Dissolution after 15 minutes | 75.5 % |

The optimized formulation is further stressed for friability by increasing the lubricant concentration to 0.75%. Hardness, dissolution and disintegration times are not affected by increasing the lubricant concentration. However, friability is higher at 14 KN compression force. Increasing the compression force to 16 KN results in acceptable friability (0.47%).

The optimized formulation is as set out in Table 11, with information about as percentage w/w, mg/dose, and kg/ 50,000 tablet batch. Based on data from development batches, minor changes in excipient concentrations will not affect the overall product attributes.

**Table 11 Optimized formulation composition**

| **% w/w** | **Ingredient** | **Mg/dose** | **Kg/batch** |
|---|---|---|---|
| | Granulation | | |
| 65.04 | COX189 Drug substance | 400.00 | 20.00 |
| 2.15 | Croscarmellose sodium, NF (Ac-Di-Sol) | 13.22 | 0.661 |
| 6.60 | Povidone K30, USP | 40.59 | 2.029 |
| 18.12 | Purified water, USP* | Qs | Qs |
| | Blending | | |
| 23.56 | Microcrystalline Cellulose, NF (Avicel PH 102) | 144.90 | 6.066 |
| 2.15 | Croscarmellose sodium, NF(Ac-Di-Sol) | 13.22 | 0.553 |
| 0.50 | Magnesium Stearate, NF (vegetable source) | 3.07 | 0.128 |
| | Film Coating | | |
| 84.46 | Opadry, Global White 00F18296 | 15.2028 | 0.296637 |
| 14.03 | Opadry, Global Red 00F15613 | 2.5254 | 0.049275 |
| 1.51 | Opadry, Global Black 00F17713 | 0.2718 | 0.005303 |
| | Purified Water, USP* | Qs | 1.990218 |
| | Film Coated Tablet Weight | 633.00 | |

| | | | |
|---|---|---|---|
| *Does not appear in final product. Percentage of water added used for granulation based on the dry weight of drug substance and croscarmellose sodium. | | | |

The batch is granulated as described above for development batches. The granulation is dried to residual moisture (% LOD) of 1.79%. The formulation process is the same as for the development batches as described above, except for the additional step of coating with Opadry in a coating pan. The coating powders (Opadry) are mixed with purified water to make a 15 % w/w coating suspension. The tablets are film coated with the coating suspension in a coating pan using 60°C to 75°C inlet air temperature. Based on friability data, a target force of 18 KN (16-20 KN range) is used to compress the remainder of the batch, resulting in acceptable friability (less than 0.5%) and the disintegration times of less than 5 mins. The ejection force is approximately 800 N throughout the compression run. This demonstrates that the blend is lubricated adequately. No picking/sticking is observed on the punch surfaces after 225 minutes. Thus, a smaller size tablet with high drug loading (65%) is achieved using a high shear granulation process, using 17 X 6.7 mm ovaloid tooling to get tablets with acceptable hardness and friability characteristics.

In addition, the tablet formulations may contain 5-methyl-2-(2'-chloro-6'-fluoroanilino)benzyl alcohol and/or 5-methyl-2-(2'-chloro-6'-fluoroanilino)benzoic acid in an amount between about 0.01 and 2% by weight, more specifically between about 0.1 and 1%. Thus, in a further embodiment the present invention is directed to a pharmaceutical composition comprising an effective amount of 5-methyl-2-(2'-chloro-6'-fluoroanilino)phenylacetic acid and between 0.01 and 2% by weight of 5-methyl-2-(2'-chloro-6'-fluoroanilino)benzyl alcohol.

## Claims

1. Use of 5-methyl-2-(2'-chloro-6'-fluoroanilino)phenylacetic acid or a pharmaceutically acceptable salt thereof in the manufacture of a medicament for treating a cyclooxygenase-2 dependent disorder or condition wherein the medicament is for once daily administration.

2. Use according to claim 1 wherein the medicament is an immediate release pharmaceutical composition.

3. Use according to claim 1 or 2 wherein the medicament is for the treatment of pain, fever, inflammation, symptoms associated with viral infections, common cold, low back and neck pain, dysmenorrhea, headache, toothache, sprains and strains, myositis, neuralgia; synovitis, arthiritis, degenerative joint diseases, gout, ankylosing spondylitis, bursitis, burns and injuries following surgical or dental procedures.

4. Use according to any one of the preceding claims wherein the medicament is a composition comprising between 60% to 90% by weight of 5-methyl-2-(2'-chloro-6'-fluoroanilino)phenylacetic acid or pharmaceutically acceptable salt thereof.

5. Use according to any one of the preceding claims wherein the medicament is a composition comprising between 50 and 400 mg of said 5-methyl-2-(2'-chloro-6'-fluoroanilino)phenylacetic acid or pharmaceutically acceptable salt thereof.

6. Use according to any one of the preceding claims wherein the medicament is a tablet composition.

7. A medicament package comprising:
(a) An immediate release pharmaceutical composition comprising 5-methyl-2-(2'-chloro-6'-fluoroanilino)phenylacetic acid or a pharmaceutically acceptable salt thereof, wherein the immediate release composition comprises an effective amount of 5-methyl-2-(2'-chloro-6'-fluoroanilino)phenylacetic acid to treat a cyclooxygenase-2 dependent disorder for about 24 hours; and
(b) printed instructions directing that the pharmaceutical composition comprising 5-methyl-2-(2'-chloro-6'-fluoroanilino)phenylacetic acid be administered orally once a day.

8. A medicament package according to claim 7 wherein the composition is for the treatment of pain, fever, inflammation, symptoms associated with viral infections, common cold, low back and neck pain, dysmenorrhea, headache, toothache, sprains and strains, myositis, neuralgia, synovitis, arthiritis, degenerative joint diseases, gout, ankylosing spondylitis, bursitis, burns and injuries following surgical or dental procedures.

9. A medicament package according to claim 7 or 8 wherein the composition comprises between 60% to 90% by weight of 5-methyl-2-(2'-chloro-6'-fluoroanilino)phenylacetic acid or pharmaceutically acceptable salt thereof.

10. A medicament package according to claim 7, 8 or 9 wherein the composition comprises between 50 and 400 mg of said 5-methyl-2-(2'-chloro-6'-fluoroanilino)phenylacetic acid or pharmaceutically acceptable salt thereof.

11. A medicament package according to any one of claims 7-10 wherein the composition is a tablet.

## Patentansprüche

1. Verwendung von 5-Methyl-2-(2'-chlor-6'-fluoranilino)phenylessigsäure oder eines pharmazeutisch akzeptablen Salzes hiervon zur Herstellung eines Arzneimittels für die Behandlung einer von Cyclooxygenase-2 abhängigen Störung oder eines davon abhängigen Zustands, wobei dieses Arzneimittel für eine einmal tägliche Verabreichung ist.

2. Verwendung nach Anspruch 1, worin das Arzneimittel eine unmittelbar freisetzende pharmazeutische Zusammensetzung ist.

3. Verwendung nach Anspruch 1 oder 2, worin das Arzneimittel ist für die Behandlung von Schmerz, Fieber, Inflammation, Symptomen, die assoziiert sind mit Vireninfektionen, gewöhnlicher Erkältung, Kreuz- und Nackenschmerz, Dysmenorrhoe, Kopfweh, Zahnweh, Verstauchungen und Zerrungen, Myositis, Neuralgie, Synovitis, Arthritis, degenerativen Gelenkkrankheiten, Gicht, ankylosierender Spondylitis, Bursitis, Verbrennungen und Schädigungen infolge chirurgischer oder dentaler Verfahren.

4. Verwendung nach einem der vorhergehenden Ansprüche, worin das Arzneimittel eine Zusammensetzung ist, die zwischen 60 Gew.-% und 90 Gew.-% 5-Methyl-2-(2'-chlor-6'-fluoranilino)phenylessigsäure oder eines pharmazeutisch akzeptablen Salzes hiervon umfasst.

5. Verfahren nach einem der vorhergehenden Ansprüche, worin das Arzneimittel eine Zusammensetzung ist, die zwischen 50 mg und 400 mg 5-Methyl-2-(2'-chlor-6'-fluoranilino)phenylessigsäure oder eines pharmazeutisch akzeptablen Salzes hiervon umfasst.

6. Verfahren nach einem der vorhergehenden Ansprüche, worin das Arzneimittel eine Tablettenzusammensetzung ist.

7. Arzneimittelpackung, umfassend
(a) eine unmittelbar freisetzende pharmazeutische Zusammensetzung, die 5-Methyl-2-(2'-chlor-6'-fluoranilino)phenylessigsäure oder ein pharmazeutisch akzeptables Salz hiervon umfasst, worin die unmittelbar freisetzende Zusammensetzung eine wirksame Menge an 5-Methyl-2-(2'-chlor-6'-fluoranilino)phenylessigsäure umfasst, zur Behandlung einer von Cyclooxygenase-2 abhängigen Störung während etwa 24 h, und
(b) Gebrauchsanweisungen, Gebrauchsinstruktionen dahingehend, dass die pharmazeutische Zusammensetzung, welche 5-Methyl-2-(2'-chlor-6'-fluoranilino)phenylessigsäure umfasst, einmal täglich verabreicht werden soll.

8. Arzneimittelpackung nach Anspruch 7, worin die Zusammensetzung ist für die Behandlung von Schmerz, Fieber, Inflammation, Symptomen, die assoziiert sind mit Vireninfektionen, gewöhnlicher Erkältung, Kreuz- und Nackenschmerz, Dysmenorrhoe, Kopfweh, Zahnweh, Verstauchungen und Zerrungen, Myositis, Neuralgie, Synovitis, Arthritis, degenerativen Gelenkkrankheiten, Gicht, ankylosierender Spondylitis, Bursitis, Verbrennungen und Schädigungen infolge chirurgischer oder dentaler Verfahren.

9. Arzneimittelpackung nach Anspruch 7 oder 8, worin die Zusammensetzung zwischen 60 Gew.-% und 90 Gew.-% 5-Methyl-2-(2'-chlor-6'-fluoranilino)phenylessigsäure oder eines pharmazeutisch akzeptablen Salzes hiervon umfasst.

10. Arzneimittelpackung nach Anspruch 7, 8 oder 9, worin die Zusammensetzung zwischen 50 mg und 400 mg 5-Methyl-2-(2'-chlor-6'-fluoranilino)phenylessigsäure oder eines pharmazeutisch akzeptablen Salzes hiervon umfasst.

11. Verfahren nach einem der Ansprüche 7 bis 10, worin die Zusammensetzung eine Tablette ist.

## Revendications

1. Utilisation d'acide 5-méthyl-2-(2'-chloro-6'-fluoroanilino) phénylacétique ou de l'un de ses sels acceptables sur le plan pharmaceutique, dans la fabrication d'un médicament pour traiter un trouble ou un état induit par la cyclooxygénase-2, le médicament étant destiné à une administration uniquotidienne.

2. Utilisation selon la revendication 1, le médicament étant une composition pharmaceutique à libération immédiate.

3. Utilisation selon la revendication 1 ou 2, le médicament étant destiné au traitement de la douleur, de la fièvre, de l'inflammation, des symptômes associés à des infections virales, du rhume banal, des lombalgies et des cervicalgies, des dysménorrhées, des céphalées, du mal de dents, des entorses et des foulures, de la myosite, des névralgies, de la synovite, de l'arthrite, de l'arthrose, de la goutte, de la spondylarthrite ankylosante, de la bursite, des brûlures et des blessures consécutives à des interventions chirurgicales ou dentaires.

4. Utilisation selon l'une quelconque des revendications précédentes, le médicament étant une composition comprenant entre 60 % et 90 % en poids d'acide 5-méthyl-2-(2'-chloro-6'-fluoroanilino)phénylacétique ou de l'un de ses sels acceptables sur le plan pharmaceutique.

5. Utilisation selon l'une quelconque des revendications précédentes, le médicament étant une composition comprenant entre 50 et 400 mg dudit acide 5-méthyl-2-(2'-chloro-6'-fluoroanilino)phénylacétique ou de l'un de ses sels acceptables sur le plan pharmaceutique.

6. Utilisation selon l'une quelconque des revendications précédentes, le médicament étant une composition pour comprimés.

7. Emballage pour médicament comprenant :
(a) une composition pharmaceutique à libération immédiate comprenant de l'acide 5-méthyl-2-(2'-chloro-6'-fluoroanilino)phénylacétique ou l'un de ses sels acceptables sur le plan pharmaceutique, la composition à libération immédiate comprenant une quantité efficace d'acide 5-méthyl-2-(2'-chloro-6'-fluoroanilino)phénylacétique pour traiter un trouble induit par la cyclooxygénase-2, pendant environ 24 heures ; et
(b) des instructions écrites informant que la composition pharmaceutique comprenant de l'acide 5-méthyl-2-(2'-chloro-6'-fluoroanilino)phénylacétique doit être administrée par voie orale une fois par jour.

8. Emballage pour médicament selon la revendication 7, la composition étant destinée au traitement de la douleur, de la fièvre, de l'inflammation, des symptômes associés à des infections virales, du rhume banal, des lombalgies et des cervicalgies, des dysménorrhées, des céphalées, du mal de dents, des entorses et des foulures, de la myosite, des névralgies, de la synovite, de l'arthrite, de l'arthrose, de la goutte, de la spondylarthrite ankylosante, de la bursite, des brûlures et des blessures consécutives à des interventions chirurgicales ou dentaires.

9. Emballage pour médicament selon la revendications 7 ou 8, la composition comprenant entre 60 % et 90 % en poids d'acide 5-méthyl-2-(2'-chloro-6'-fluoroanilino)phénylacétique ou de l'un de ses sels acceptables sur le plan pharmaceutique.

10. Emballage pour médicament selon la revendication 7, 8 ou 9, la composition comprenant entre 50 et 400 mg dudit acide 5-méthyl-2-(2'-chloro-6'-fluoroanilino)phénylacétique ou de l'un de ses sels acceptables sur le plan pharmaceutique.

11. Emballage pour médicament selon l'une quelconque des revendications 7 à 10, le médicament étant un comprimé.
